# EUROPÄISCHE PATENTSCHRIFT

(11) **EP 2 595 691 B1**
(45) Veröffentlichungstag und Bekanntmachung des Hinweises auf die Patenterteilung: **20.03.2019**
(21) Anmeldenummer: 11741517.4
(22) Anmeldetag: 22.07.2011
(51) Int. Cl.: A61M 39/10, A61M 39/16, A61M 39/18, A61M 39/20, A61M 39/00, A61M 1/00, A61M 1/16, A61M 1/36

(54) **KONNEKTOR, MEDIZINTECHNISCHE BEHANDLUNGSVORRICHTUNG, MEDIZINTECHNISCHE FUNKTIONSEINRICHTUNG SOWIE VERFAHREN**
CONNECTOR, MEDICAL TREATMENT DEVICE, MEDICAL FUNCTIONAL DEVICE AND METHOD
CONNECTEUR, DISPOSITIF DE TRAITEMENT DE TECHNIQUE MÉDICALE, DISPOSITIF FONCTIONNEL DE TECHNIQUE MÉDICALE ET PROCÉDÉ

(30) Priorität: 23.07.2010 DE 102010032179
(43) Veröffentlichungstag der Anmeldung: 29.05.2013
(73) Patentinhaber: Fresenius Medical Care Deutschland GmbH, 61352 Bad Homburg (DE)
(72) Erfinder: HAECKER, Juergen, 61267 Neu-Anspach (DE); NOACK, Joachim, 97616 Bad Neustadt (DE); LAPP, Uwe, 35510 Butzbach (DE); KOEHLER, Markus, 61440 Oberursel (DE); WIESEN, Gerhard, 61352 Bad Homburg (DE); GUENTHER, Goetz, verstorben (DE)
(74) Vertreter: Bobbert & Partner Patentanwälte PartmbB
(86) Internationale Anmeldenummer: PCT/EP2011/003684
(87) Internationale Veröffentlichungsnummer: WO 2012/010322

(56) Entgegenhaltungen:
- EP-A2- 1 279 409
- WO-A1-83/00447
- WO-A1-2008/028579
- WO-A1-2010/127461
- WO-A2-2010/121751
- US-A- 4 209 013
- US-A1- 2009 137 944

## Beschreibung

Die vorliegende Erfindung betrifft einen Konnektor gemäß Anspruch 1.

Das Verbinden oder Konnektieren medizintechnischer Systeme zum Herstellen einer Fluidverbindung zwischen zwei solcher Systeme ist aus der Praxis bekannt.

WO 83/00447 A1 und US 4 209 013 A offenbaren Verbindungssysteme zwischen einer Patientenkavität und ein Lösungsbehälter. EP 1 279 409 A2 offenbart einen zwischen zwei Positionen bewegbaren Konnektor zur Verbindung einer Losungsmittelquelle mit einem ersten und einem zweiten Konzentratbehältnis. US 2009/137944 A1 offenbart einen Konnektor zur Anbringung an einem Katheter. WO 2008/028579 A1 offenbart ein Verfahren zum Entleeren eines Blutschlauchsatzes.

Eine Aufgabe der vorliegenden Erfindung ist, einen zu diesem Zweck geeigneten Konnektor anzugeben, der dazu geeignet ist, vor dem Anschließen an eine Funktionseinrichtung, mittels Sterilluft geleert zu werden, so dass die zur Fluidverbindung vorgesehenen Fluidführungen und Kontaktflächen des Konnektors von möglichen Keimen gereiningt sind.

Die erfindungsgemäße Aufgabe wird durch einen Konnektor mit den Merkmalen des Anspruchs 1 gelöst.

Alle oder manche mit dem erfindungsgemäßen Konnektor erzielbaren Vorteile lassen sich unter Zuhilfenahme der mit der medizintechnischen Behandlungsvorrichtung und/oder der medizintechnischen Funktionseinrichtung und/oder dem Verfahren und/oder der Steuereinrichtung erzielen.

Erfindungsgemäß wird ein Konnektor vorgeschlagen, der zum Herstellen einer Fluidverbindung zwischen wenigstens zwei medizintechnischen Systemen geeignet und vorgesehen ist.

Der erfindungsgemäße Konnektor weist einen mehrlumigen Endabschnitt mit einer ersten Fluidführung mit wenigstens einem ersten Lumen auf, welches vorgesehen ist zur Aufnahme und/oder Führung eines ersten, medizinischen, Fluids.

Ferner weist der mehrlumige Endabschnitt des erfindungsgemäßen Konnektors eine zweite Fluidführung mit wenigstens einem zweiten Lumen auf, welches vorgesehen ist zur Aufnahme und/oder Führung eines zweiten, vom ersten verschiedenen, Fluids.

Die medizintechnische Behandlungsvorrichtung (im Folgenden auch kurz: Behandlungsvorrichtung) und die medizintechnische Funktionseinrichtung (im Folgenden auch kurz: Funktionseinrichtung) sind jeweils vorgesehen zur Verbindung mit, oder verbunden mit, wenigstens einem Konnektor gemäß der vorliegenden Erfindung.

Vorteilhafte Weiterbildungen der vorliegenden Erfindung sind jeweils Gegenstand der Unteransprüche.

Erfindungsgemäße Ausführungsformen können einige oder alle der folgenden Merkmale in beliebiger Kombination aufweisen. Der Begriff "Fluidverbindung", wie er hierin verwendet wird, bezeichnet in bestimmten Ausführungsformen der vorliegenden Erfindung eine mittels eines Konnektors oder Verbinders erzeugte, i.a.R. vorübergehende, Verbindung, die dem Führen bzw. Leiten und/oder Überführen bzw. Übertragen und dergleichen von Fluiden zwischen zwei fluidführenden Systemen, z.B. einer Funktionseinrichtung als einem ersten System und einer Behandlungsvorrichtung als einem zweiten System, dient.

Dabei kann der erfindungsgemäße Konnektor in bestimmten Ausführungsformen der vorliegenden Erfindung Teil eines der beiden medizintechnischen Systeme, z.B. der medizintechnischen Behandlungsvorrichtung, sein.

Der Begriff "mehrlumiger Endabschnitt", wie er hierin verwendet wird, bezeichnet einen an einem Ende gelegenen Abschnitt des Konnektors in einem Verbindungs- bzw. Konnektionsbereich des Konnektors, in welchem Lumen mit separaten Austrittsöffnungen vorliegen.

Der Begriff "Fluidführung", wie er hierin verwendet wird, bezeichnet in bestimmten Ausführungsformen der vorliegenden Erfindung allgemein eine physische bzw. körperliche Anordnung von Elementen, welche zum Aufnehmen und/oder Leiten, Führen und dergleichen von Fluiden vorgesehen sind. Beispiele hierfür sind Rohre, Schläuche, Kanäle, Leitungen, Kammern, Fluidführungseinrichtungen, usw.

In bestimmten Ausführungsformen der vorliegenden Erfindung ist die erste Fluidführung vorgesehen zum Ausleiten bzw. Austragen des ersten, medizinischen, Fluids aus einer Austrittsöffnung der ersten Fluidführung (also aus dem Konnektor) heraus, z.B. in die Umgebung, in ein Äußeres, in die verbundene Funktionseinrichtung, usw.

Das erste, medizinische, Fluid ist dabei in bestimmten Ausführungsformen dasjenige Fluid, das über die Fluidverbindung von einem System auf das andere übertreten bzw. übertragen werden soll.

In bestimmten Ausführungsformen der vorliegenden Erfindung ist vorgesehen, das erste, medizinische, Fluid durch die Austrittsöffnung oder ein freies Ende der ersten Fluidführung - direkt aus der ersten Fluidführung - in ein Inneres einer Fluidführung des zur Fluidverbindung mit dem Konnektor verbundenen medizintechnischen Systems, z.B. einer Funktionseinrichtung, eintreten zu lassen.

In bestimmten Ausführungsformen der vorliegenden Erfindung kann vorgesehen sein, dass das erste medizinische Fluid während eines Füll- und/oder Spül- und/oder Primingprozesses und/oder während eines Behandlungsverfahrens durch das erste Lumen der ersten Fluidführung in die Funktionseinrichtung strömt.

Der Begriff "medizinisches Fluid", wie er hierin verwendet wird, bezeichnet in bestimmten Ausführungsformen der vorliegenden Erfindung allgemein Flüssigkeiten, wie Dialysat, Substituatflüssigkeit, Medikamentenlösungen, Priming-und/oder Spül- und/oder Sterilisationsfluide und dergleichen sowie Gase, z.B. Sterilluft, sowie beliebige Kombinationen oder Mischungen derselben und mit denselben. Das medizinische Fluid ist in bestimmten Ausführungsformen der vorliegenden Erfindung für eine extrakorporale Blutbehandlung geeignet und/oder vorgesehen oder bestimmt.

In bestimmten Ausführungsformen der vorliegenden Erfindung ist das medizinische Fluid Substituatflüssigkeit, in manchen erfindungsgemäßen Ausführungsformen von einer Behandlungsvorrichtung online hergestellte Substituatflüssigkeit.

In bestimmten Ausführungsformen der vorliegenden Erfindung ist die zweite Fluidführung vorgesehen zum Ausleiten bzw. Austragen des zweiten Fluids aus einer Austrittsöffnung der zweiten Fluidführung (also aus dem Konnektor) heraus.

In manchen Ausführungsformen der vorliegenden Erfindung ist vorgesehen, das aus der Austrittsöffnung oder einem freien Ende der zweiten Fluidführung austretende zweite Fluid in ein Äußeres des Konnektors austreten zu lassen.

In bestimmten Ausführungsformen der vorliegenden Erfindung weist die zweite Fluidführung an einer - von der Austrittsöffnung im Bereich des mehrlumigen Endabschnitts des Konnektors verschiedenen Stelle - wenigstens eine Eintrittsöffnung auf, in bzw. durch welche das zweite Fluid in das zweite Lumen der zweiten Fluidführung eingebracht werden kann.

Das zweite Fluid ist dabei in bestimmten Ausführungsformen ein Fluid, das nicht über die Fluidverbindung von einem System auf das andere übertreten bzw. übertragen werden soll. Das zweite Fluid kann ein - vom ersten medizinischen Fluid verschiedenes - medizinisches Fluid sein.

Beispiele für das zweite Fluid schließen, ohne darauf beschränkt zu sein, Gase, wie Sterilluft und dergleichen, ein.

In der vorliegenden Erfindung weist der Konnektor wenigstens eine dritte Fluidführung auf. Die dritte Fluidführung weist wenigstens ein drittes Lumen auf, welches vorgesehen ist zur Aufnahme und/oder Führung des ersten und/oder des zweiten Fluids.

In bestimmten Ausführungsformen der vorliegenden Erfindung ist das zweite Lumen oder dessen Austrittsöffnung oder dessen freies Ende in einem oberen Bereich des Konnektors angeordnet und das dritte Lumen bzw. eine Austrittsöffnung desselben in einem unteren Bereich des Konnektors.

Die Begriffe "oberer Bereich" und "unterer Bereich" sind Lagebezeichnungen, welche sich auf eine Anordnung des Konnektors in einer normalen Gebrauchsstellung des Konnektors beziehen.

Ein "oberer Bereich" im Sinne der Erfindung ist in manchen Ausführungsformen jeder Bereich, welcher (bezogen auf eine Vertikale) oberhalb des "unteren Bereichs" liegt. Ebenso ist ein "unterer Bereich" in diesen Ausführungsformen ein Bereich, welcher (bezogen auf eine Vertikale) unterhalb des "oberen Bereichs" liegt.

In bestimmten Ausführungsformen der vorliegenden Erfindung ist die zweite Fluidführung in einem Gebrauchszustand des Konnektors oberhalb der ersten Fluidführung angeordnet.

In bestimmten Ausführungsformen der vorliegenden Erfindung ist die dritte Fluidführung in einem Gebrauchszustand des Konnektors unterhalb der ersten und/oder der zweiten Fluidführung angeordnet.

In Ausführungsformen, in denen die Austrittsöffnung oder das freie Ende des zweiten Lumens der zweiten Fluidführung oben angeordnet ist und/oder die Austrittsöffnung oder das freie Ende des dritten Lumens der dritten Fluidführung unten angeordnet ist, kann es vorteilhaft möglich sein, einen Austritt des zweiten Fluids (aus der zweiten Fluidführung) bzw. des zweiten und/oder des ersten Fluids (aus der dritten Fluidführung) durch Wirkung der Schwerkraft zu begünstigen.

Eine Durchströmungsrichtung des ersten Lumens und eine Durchströmungsrichtung des zweiten Lumens und eine Durchströmungsrichtung des dritten Lumens können gleich oder verschieden sein.

Der Begriff "Durchströmungsrichtung", wie er hierin verwendet wird, bezeichnet eine Richtung, in welcher ein Fluid ein Lumen des Konnektors durchströmt.

Die Durchströmungsrichtung ist die Richtung, in welcher vorgesehen ist, dass die Fluide im bestimmungsgemäßen Gebrauch des Erfindungsgegenstandes durch den Konnektor strömen sollen oder werden.

In manchen Ausführungsformen der vorliegenden Erfindung sind die Durchströmungsrichtung des ersten Lumens und die Durchströmungsrichtung des zweiten Lumens im Wesentlichen oder vollständig gleich oder identisch.

In bestimmten Ausführungsformen der vorliegenden Erfindung strömen das erste, medizinische, Fluid und das zweite Fluid in der gleichen Richtung in und durch den Konnektor und strömen in der entgegengesetzten Richtung durch das dritte Lumen aus dem Konnektor heraus.

In bestimmten Ausführungsformen der vorliegenden Erfindung wird das zweite Fluid, z.B. Sterilluft, durch das freie Ende der zweiten Fluidführung zu einem Äußeren des Konnektors geleitet. Von dort wird das zweite Fluid - durch eine Eintrittsöffnung oder ein freies Ende der dritten Fluidführung - in die dritte Fluidführung hinein geleitet oder von dieser ungeleitet aufgenommen. Aus der dritten Fluidführung wird das zweite Fluid abgeleitet, entsorgt oder dergleichen.

In bestimmten Ausführungsformen der vorliegenden Erfindung ist die zweite Fluidführung als Zuleitung für das zweite Fluid und die dritte Fluidführung als Ableitung für das erste, medizinische, Fluid und/oder das zweite Fluid vorgesehen und ausgestaltet.

In solchen Ausführungsformen der vorliegenden Erfindung können das zweite und das dritte Lumen des Konnektors zum Reinigen und/oder Leeren des Konnektors vor seiner Verwendung dienen.

In bestimmten Ausführungsformen der vorliegenden Erfindung durchströmen das erste, medizinische, Fluid und das zweite Fluid den Konnektor zur gleichen Zeit. In anderen Ausführungsformen durchströmen das erste Fluid und das zweite Fluid den Konnektor zu verschiedenen Zeitpunkten oder Zeiträumen.

In der vorliegenden Erfindung ist der erfindungsgemäße Konnektor vorgesehen und/oder ausgestaltet, um in einem Verschlusszustand im Bereich seines Endabschnitts mit einer Spülkappe verschlossen zu werden. Eine solche Spülkappe kann vorzugsweise als Verschlusskappe ausgeführt sein.

In einer ersten Stellung bzw. einem ersten Zustand oder Grundzustand kann der mit einer Spülkappe verschlossene Konnektor im Wesentlichen oder vollständig mit dem ersten Fluid gefüllt sein.

In bestimmten Ausführungsformen ist vorgesehen, das zweite Fluid zum Austragen des ersten Fluids aus dem Konnektor und/oder aus einem Bereich um den Konnektor herum einzusetzen.

Der Begriff "Verschlusszustand", wie er hierin verwendet wird, bezeichnet in bestimmten Ausführungsformen der vorliegenden Erfindung einen Zustand, in welchem der Konnektor nicht an beiden Seiten mit je einem medizintechnischen System konnektiert ist. In bestimmten Ausführungsformen der vorliegenden Erfindung bezeichnet ein Verschlusszustand einen Zustand, in welchem der erfindungsgemäße Konnektor nicht mit einer Funktionseinrichtung konnektiert ist. In solchen Ausführungsformen kann der Konnektor z.B. nur mit der Behandlungsvorrichtung konnektiert sein oder als fest integrierter Bestandteil der Behandlungsvorrichtung an dieser angeordnet sein.

Eine "Spülkappe", wie sie in der vorliegenden Erfindung eingesetzt wird, bezeichnet eine Verschlusseinrichtung, welche dazu ausgelegt und/oder vorgesehen ist, den Konnektor gegenüber einem Äußeren des Konnektors zu verschließen oder abzudichten. Es kann sich vorzugsweise um eine Verschlusskappe handeln.

Die Spülkappe verschließt oder dichtet den erfindungsgemäßen Konnektor gegen ein Äußeres ab. Die Lumen des Konnektors stehen mit einem Inneren der Spülkappe in Fluidkontakt.

In bestimmten Ausführungsformen der vorliegenden Erfindung ist die Spülkappe des Konnektors so ausgeführt, dass sie in einer Spülstellung zur Außenseite oder zu einem Äußeren der medizintechnischen Behandlungsvorrichtung hin abdichtet.

Die Spülkappe ist in bestimmten Ausführungsformen der vorliegenden Erfindung dazu vorgesehen, eine Reinigung des Konnektors zu ermöglichen oder zu begünstigen.

In bestimmten Ausführungsformen ist die Spülkappe lösbar mit dem Konnektor verbindbar ausgestaltet. Die Spülkappe kann beispielsweise auf den Konnektor bzw. den mehrlumigen Endabschnitt des Konnektors aufschraubbar oder aufsteckbar oder aufclipbar, an den Konnektor anflanschbar und dergleichen sein.

In bestimmten Ausführungsformen der vorliegenden Erfindung ist vorgesehen, den Konnektor durch Herausfahren des Konnektors aus der Spülkappe zu öffnen. Umgekehrt ist es in manchen erfindungsgemäßen Ausführungsformen auch möglich, eine verfahrbare Spülkappe von einem feststehenden Konnektor wegzufahren. Das Öffnen des Konnektors kann zum Zwecke des Verbindens des Konnektors mit einem medizintechnischen System, z.B. der Funktionseinrichtung, erfolgen.

In bestimmten Ausführungsformen ist die Spülkappe vom Konnektor bzw. dem mehrlumigen Endabschnitt desselben verschwenkbar angeordnet.

Die Spülkappe kann automatisch oder automatisiert verschwenkbar und/oder verschiebbar ausgestaltet sein.

Die Spülkappe kann derart am Konnektor bzw. am Endabschnitt des Konnektors angeordnet oder zur Anordnung vorgesehen sein, dass im Zustand der aufgesetzten Spülkappe wenigstens ein Abschnitt der Spülkappe zwischen einem ersten Teil und einem zweiten Teil des Konnektors angeordnet ist. Bei dem wenigstens einen Abschnitt der Spülkappe kann es sich um einen Dichtungsumgriff handeln.

Dieser Abschnitt der Spülkappe kann zwischen einem radial weiter außen gelegenen Teil des Konnektors und einem radial weiter innen gelegenen Teil des Konnektors angeordnet sein.

In bestimmten Ausführungsformen der vorliegenden Erfindung ist die Spülkappe derart am Konnektor angeordnet, dass ein radial weiter außen gelegener Teil des Konnektors eine Außenseite der Spülkappe umgreift.

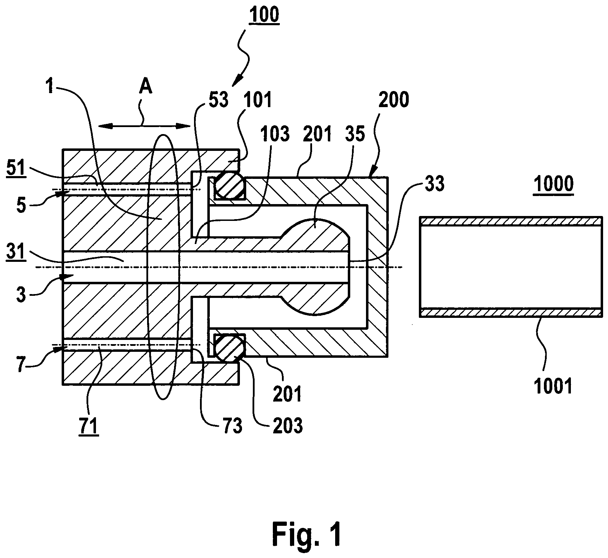

Ein Beispiel für eine solche Anordnung ist in Fig. 1 gezeigt.

Der radial weiter außen gelegene Teil des Konnektors kann in Berührkontakt mit der Außenseite der Spülkappe stehen.

In bestimmten Ausführungsformen der vorliegenden Erfindung weist die Spülkappe Dichtflächen auf.

In bestimmten Ausführungsformen der vorliegenden Erfindung sind die Dichtflächen der Spülkappe an der Außenseite derselben angeordnet. Dies kann in bestimmten Ausführungsformen vorteilhaft sein, um eine möglichst restlose bzw. rückstandsfreie Leerung des Konnektors zu erreichen.

In bestimmten Ausführungsformen der vorliegenden Erfindung, wie beispielsweise auch in Fig. 1 gezeigt, können eine oder mehrere Dichteinrichtungen, an der Außenseite der Spülkappe oder zwischen der Spülkappe und einer Innenseite des radial weiter außen gelegenen Teils des Konnektors angeordnet sein, jedenfalls im Verbindungszustand zwischen Konnektor und Spülkappe.

In der vorliegenden Erfindung ist die Spülkappe derart am Konnektor angeordnet, dass das zweite Lumen der zweiten Fluidführung in Fluidkontakt mit einem Inneren der Spülkappe steht.

In solchen Ausführungsformen kann das zweite Fluid aus der Austrittsöffnung oder dem freien Ende des zweiten Lumens aus dem Konnektor heraus und in das Innere der Spülkappe eingebracht werden.

In der vorliegenden Erfindung steht die erste Fluidführung in Fluidkontakt mit dem Inneren der Spülkappe. Dies kann insbesondere in Ausführungsformen gewünscht und/oder erforderlich sein, in denen ein Spülen bzw. Reinigen des Konnektors vorgesehen ist.

In der vorliegenden Erfindung steht die dritte Fluidführung in Fluidkontakt mit dem Inneren der Spülkappe.

In der vorliegenden Erfindung ist es damit möglich, Fluid (das erste und/oder das zweite Fluid) aus dem Inneren der Spülkappe über das dritte Lumen aus dem Konnektor abzuleiten. In bestimmten Ausführungsformen der vorliegenden Erfindung ist vorgesehen, einen Fluidzutritt aus dem Inneren der Spülkappe in das erste Lumen zu verhindern. Geeignete Einrichtungen hierfür, wie beispielsweise Einrichtungen zum Erzeugen eines Überdrucks oder erhöhten Drucks im ersten Lumen, können vorgesehen sein. Diese Einrichtungen können Ventile umfassen.

In manchen Ausführungsformen ist vorgesehen, mittels einer Ultrafiltrationspumpe oder einer anderen Pumpe einen Unterdruck zu erzeugen. Der Unterdruck kann in einem Bereich zwischen einem Ventil einer Drain-Leitung, dem Konnektor selbst und einem Ventil der dritten Fluidführung erzeugt werden. Ein Ventil der Sterilluftzuleitung wird zumindest für kurze Zeit geöffnet. Das Ventil der dritten Fluidführung kann dabei geschlossen und anschließend erneut geöffnet sein oder werden. Dieses Vorgehen kann einmal oder mehrfach ausgeführt werden. Der Konnektor kann schließlich bei anliegendem Unterdruck geöffnet werden.

In solchen Ausführungsformen kann vorgesehen sein, einen Unterdruck nicht über einen pneumatischen Anschluss oder ein pneumatisches System, und/oder nicht mit Hilfe eines an einem pneumatischen Anschluss angeschlossenen Kompressors zu erzeugen.

Ein pneumatisches System, wie hierin verwendet, weist in einigen erfindungsgemäßen Ausführungsformen eine Luftverteilerplatte auf.

Das Erzeugen des Unterdrucks mittels der Ultrafiltrationspumpe oder der anderen Pumpe kann vorteilhaft zu einem möglichst schonenden Einbringen von Luft beitragen. Anders als beim Einblasen unter Druck kann ggf. ein Auseinanderreißen der Flüssigkeit, wobei einzelne Tropfen im Konnektor verbleiben können, vermieden werden. Derartige Tröpfchen können ggf. nicht mehr abgesaugt werden. Das Öffnen bei anstehendem Unterdruck kann vorteilhaft dazu beitragen, etwaige Tropfen aus dem Dichtungsbereich nicht nach außen gelangen zu lassen, sondern durch die aufgrund des Unterdrucks einströmende Luft nach innen mitzureißen. Auf diese Weise sind optimale Leerungsergebnisse möglich.

In manchen Ausführungsformen steht oder ragt die sterile Konnektorspitze über Öffnungen oder freie Enden der zweiten Fluidführung, der dritten Fluidführung und/oder einen Dichtring über. Wird daher eine Luftströmung, welche beim Öffnen der Spülkappe aufgrund des Druckausgleichs entsteht oder vorliegt, von der Eintrittstelle der Luftströmung, welche im Bereich des Dichtrings liegt, mittels Unterdruck zur Öffnung oder zum freien Ende der zweiten und/oder der dritten Fluidführung geleitet, so kann von außen eintretende Luft, welche Umgebungskeime aufweisen kann, nach ihrem Eintritt in das Innere der Spülkappe über die zweite und/oder dritte Fluidführung abgeführt oder abgesaugt werden. Vorteilhaft ist bei dieser Anordnung, dass der kugelförmige Endbereich des Konnektors, welchen es steril oder keimfrei zu halten gilt, jedenfalls im Moment des Öffnens der Spülkappe nicht im Strömungsweg der eintretenden Luft (dieser führt vom Dichtring zur Öffnung oder zum Endbereich der zweiten und/oder dritten Fluidführung) liegt. Vielmehr liegt der Endbereich des Konnektors in der hier beschriebenen Ausführung sowohl vor dem Dichtring, an welchem Umgebungsluft eintritt, als auch vor der Öffnung oder dem Endbereich von zweiter und/oder dritter Fluidführung, durch welche die eingetretene Umgebungsluft wegen Unterdruck wieder aus der Spülkappe austritt. Ein Verschleppen von Keimen durch eintretende Luft und/oder durch Flüssigkeit aus dem Dichtbereich kann somit vorteilhaft ausgeschlossen werden, da die eintretende Luft bei dieser Ausgestaltung nicht zum Endbereich des Konnektors gelangt.

In bestimmten Ausführungsformen ist vorgesehen, ein Material des Konnektors, insbesondere im Endabschnitt desselben, so auszuwählen, dass ein Entleerungsverhalten der Spülkappe optimiert ist.

In gewissen Ausführungsformen der vorliegenden Erfindung weist der Konnektor oder sein Endabschnitt beispielsweise PEEK (Polyetheretherketon)_mit PTFE- (Polytetrafluorethylen-) Beimischung) auf oder besteht hieraus. Diese oder eine ebenfalls vorgesehene ähnliche Materialkombination können aufgrund der guten Gleiteigenschaften vorteilhaft die zu überwindenden Konnektionskräfte an der medizintechnischen Funktionseinrichtung senken.

In bestimmten Ausführungsformen der vorliegenden Erfindung weist der erfindungsgemäße Konnektor wenigstens in bestimmten Abschnitten eine hydrophile Beschichtung auf oder ist - in diesen Abschnitten - aus einem hydrophilen Material hergestellt. In bestimmten Ausführungsformen weist der Konnektor wenigstens in manchen Abschnitten eine hydrophobe Beschichtung auf oder ist - in diesen Abschnitten - aus einem hydrophoben Material hergestellt.

Um einen Tropfenflug z.B. während des Weg- oder Verschwenkens der Spülkappe zu minimieren, kann in bestimmten Ausführungsformen der vorliegenden Erfindung eine hydrophile Beschichtung des Konnektors und/oder der Spülkappe bevorzugt sein.

In bestimmten Ausführungsformen der vorliegenden Erfindung ist der erfindungsgemäße Konnektor vorgesehen zur Verbindung mit oder verbunden mit einer medizintechnischen Blutbehandlungsvorrichtung.

In bestimmten Ausführungsformen der vorliegenden Erfindung ist der erfindungsgemäße Konnektor Teil eines maschinenseitigen Substituatsystems einer extrakorporalen Behandlungsvorrichtung.

In bestimmten Ausführungsformen ist der Konnektor integral mit der Behandlungsvorrichtung hergestellt. In anderen Ausführungsformen der vorliegenden Erfindung kann der Konnektor jedoch auch an der Behandlungsvorrichtung nach- bzw. aufgerüstet werden oder nach- bzw. aufgerüstet worden sein.

In bestimmten Ausführungsformen der vorliegenden Erfindung ist der Konnektor an der medizintechnischen Behandlungsvorrichtung zur Horizontalen geneigt angebracht.

Der Begriff "geneigt", wie er hierin verwendet wird, bezeichnet in bestimmten Ausführungsformen der vorliegenden Erfindung ein zur Horizontalen unter einem Winkel angeordnetes erstes und/oder zweites und/oder drittes Lumen des Konnektors.

In bestimmten Ausführungsformen ist der Konnektor - im Verschlusszustand - innerhalb der Spülkappe geneigt angeordnet.

Der Konnektor kann dauerhaft oder vorübergehend geneigt angeordnet sein. In bestimmten Ausführungsformen ist der Konnektor nach einem Spülen oder Reinigen des Konnektors, vor einer Behandlung und dergleichen geneigt angeordnet.

In bestimmten Ausführungsformen kann der Konnektor dabei in einem Winkelbereich von 8° ± 3° zur Horizontalen geneigt angebracht sein.

In manchen erfindungsgemäßen Ausführungsformen ist der Konnektor derart angeordnet, dass in wenigstens einer Stellung (Spülstellung, Verschlussstellung, usw.) sein erstes Lumen eine wie oben beschriebene Neigung zur Horizontalen aufweist, wobei bei dieser Neigung das freie Ende des ersten Lumens tiefer steht als andere Abschnitte hiervon.

Ebenso kann der Konnektor in bestimmten erfindungsgemäßen Ausführungsformen derart angeordnet sein, dass das dritte Lumen eine wie oben beschriebene Neigung zur Horizontalen aufweist, wobei bei dieser Neigung das freie Ende des dritten Lumen höher steht als andere Abschnitte hiervon. Diese Anordnung kann vorteilhaft ein besonders gründliches Ablaufen von Fluid, auch Fluidtropfen, aus dem Inneren der Spülkappe ermöglichen.

In bestimmten Ausführungsformen der vorliegenden Erfindung ist der erfindungsgemäße Konnektor vorgesehen zur Verbindung mit oder verbunden mit einer medizintechnischen Funktionseinrichtung.

Der erfindungsgemäße Konnektor kann direkt oder mittels einer geeigneten Einrichtung mit der Funktionseinrichtung verbindbar oder verbunden sein.

Geeignete Beispiele für solche Einrichtungen zur Verbindung des erfindungsgemäßen Konnektors mit der Funktionseinrichtung schließen solche Einrichtungen ein, die in der von der vorliegenden Anmelderin am 23. Juli 2010 unter dem Amtszeichen DE 10 2010 032 181.8 beim Deutschen Patent- und Markenamt hinterlegten Anmeldung mit dem Titel *"Ankoppeleinrichtung, Konnektor, medizintechnische Funktionseinrichtung, medizintechnische Behandlungsvorrichtung sowie Verfahren"* beschrieben sind und auf deren diesbezügliche Offenbarung hiermit vollinhaltlich Bezug genommen wird.

In bestimmten Ausführungsformen der vorliegenden Erfindung ist der erfindungsgemäße Konnektor vorgesehen, um zur Verbindung mit der medizintechnischen Funktionseinrichtung oder eines Abschnitts derselben in einer Richtung der Ankopplung zur medizintechnischen Funktionseinrichtung verschoben zu werden.

Der erfindungsgemäße Konnektor kann, vorzugsweise in Richtung seiner Längsrichtung oder in Richtung seines ersten Lumens oder in Richtung der Ankopplung, bezogen auf die Behandlungsvorrichtung verschiebbar ausgestaltet sein.

Der Begriff "Richtung der Ankopplung", wie er hierin verwendet wird, bezeichnet in bestimmten Ausführungsformen der vorliegenden Erfindung diejenige Richtung, in welcher der Konnektor zur Ankopplung der Behandlungsvorrichtung mit der Funktionseinrichtung zur Funktionseinrichtung hin bewegt wird.

Der Konnektor kann zur Funktionseinrichtung hin und von dieser weg beweglich ausgestaltet sein.

Der Konnektor kann automatisch oder automatisiert bewegt bzw. verschoben werden. Dies kann vorteilhaft dazu beitragen, dass Benutzeraktionen weiter entfallen können.

Die automatische Verschiebung des Konnektors wird in bestimmten Ausführungsformen über einen elektromotorischen Antrieb erreicht.

In bestimmten Ausführungsformen der vorliegenden Erfindung ist der Konnektor aus der Spülkappe herausfahrbar ausgestaltet.

Die erfindungsgemäße medizintechnische Behandlungsvorrichtung ist in manchen erfindungsgemäßen Ausführungsformen eines der beiden medizintechnischen Systeme, welche zur Fluidverbindung mittels des Konnektors vorgesehen sind.

Die erfindungsgemäße Behandlungsvorrichtung ist in bestimmten Ausführungsformen als extrakorporale Behandlungsvorrichtung, insbesondere als extrakorporale Blutbehandlungsvorrichtung, etwa als Dialysiervorrichtung, insbesondere als Hämodialysiervorrichtung, Hämofiltrationsvorrichtung, Hämodiafiltrationsvorrichtung, oder als Vorrichtung für die Adsorption, Leberersatztherapie, Apherese, Transfusion, usw. ausgestaltet.

Ihr Einsatz zu Behandlungszwecken ist jedoch nicht auf die hierin beispielhaft angegebenen Verwendungen beschränkt.

Die erfindungsgemäße Behandlungsvorrichtung weist in bestimmten Ausführungsformen der vorliegenden Erfindung wenigstens eine Einrichtung zum Einleiten von Luft in die zweite Fluidführung auf.

Eine "Einrichtung zum Einleiten von Luft", wie sie hierin verwendet wird, umfasst in bestimmten Ausführungsformen der Erfindung ein System bzw. eine Anordnung, welche dazu ausgelegt ist, Luft in die zweite Fluidführung einzuleiten bzw. einzubringen, wie einzublasen und/oder einzusaugen.

In bestimmten Anwendungen muss die eingeleitete Luft nicht steril sein, beispielsweise dann, wenn ein Sterilfilter für die Substitutionsflüssigkeit in der externen Funktionseinrichtung (z.B. Blutkassette) vorgesehen ist.

Die Luft ist in bestimmten Ausführungsformen bevorzugt keimarm oder steril.

In bestimmten Ausführungsformen umfasst die Einrichtung zum Einblasen von Sterilluft Mittel zum Erzeugen von Sterilluft oder steriler Pressluft aus z.B. Umgebungsluft wie wenigstens einen Filter oder Sterilfilter, einen Kompressor und dergleichen.

Die Einrichtung zum Einblasen der Sterilluft - oder ganz allgemein des zweiten Fluids - kann an einer beliebigen Stelle der zweiten Fluidführung, jedoch an einer von der Austrittsöffnung oder des freien Endes des zweiten Lumens verschiedenen Stelle, vorgesehen sein.

Die erfindungsgemäße Funktionseinrichtung ist das andere der medizintechnischen Systeme, welche zur Fluidverbindung mittels des Konnektors vorgesehen sind.

Nicht einschränkende Beispiele für Funktionseinrichtungen im Sinne der vorliegenden Erfindung schließen eine Blutkassette, einen Blutschlauchsatz und dergleichen ein. Die Funktionseinrichtung kann als extrakorporaler Blutkreislauf vorgesehen sein oder einen solchen aufweisen.

In bestimmten Ausführungsformen der vorliegenden Erfindung ist die Funktionseinrichtung als Blutkassette ausgestaltet, oder Teil einer solchen, oder weist eine solche auf. Eine solche Blutkassette kann zum Beispiel als (Kunststoff-) Gussteil oder Spritzgussteil ausgestaltet sein. Sie kann unabhängig hiervon als Einwegartikel oder Disposable ausgestaltet sein.

Beispiele für geeignete Blutkassetten schließen die in der Anmeldung DE 10 2009 018 664.6, die am 23. April 2009 beim Deutschen Patent- und Markenamt eingereicht wurde, sowie die in der Anmeldung DE 10 2009 024 468.9, die am 10. Juni 2009 beim Deutschen Patent- und Markenamt eingereicht wurde, welche jeweils den Titel "Externe Funktionseinrichtung, Blutbehandlungsvorrichtung zum Aufnehmen einer erfindungsgemäßen externen Funktionseinrichtung, sowie Verfahren" tragen, jeweils von der Anmelderin der vorliegenden Anmeldung, beschriebenen Ausführungsformen ein, ohne auf diese beschränkt zu sein.

Ein Verfahren zum Verbinden der beiden fluidführenden medizintechnischen Systeme kann verschiedene Phasen des Konnektierens beinhalten, sowie das Ausblasen des zweiten Fluids aus der zweiten Fluidführung des Konnektors.

Das Ausblasen des zweiten Fluids, z.B. Sterilluft, kann vor dem eigentlichen Herstellen der Fluidverbindung des Konnektors mit dem zweiten der medizintechnischen Systeme, z.B. der Funktionseinrichtung, erfolgen.

Das Ausblasen oder allgemein Verdrängen kann im Verschlusszustand des Konnektors erfolgen.

In bestimmten Beispielen umfasst das Verfahren das Erzeugen eines Überdrucks im Bereich des Endabschnitts des Konnektors unter Wirkung der Spülkappe.

In solchen Beispielen kann der Überdruck mittels Ausblasen des zweiten Fluids aus der zweiten Fluidführung erzeugt werden. Das Erzeugen des Überdrucks kann vor dem Öffnen der Spülkappe, z.B. durch Verschwenken der Spülkappe vom Endabschnitt des Konnektors, erfolgen.

Das zweite Fluid kann dabei aus der Austrittsöffnung des zweiten Lumens der zweiten Fluidführung zu einem Äußeren des Konnektors, welches im Verschlusszustand einem Inneren der Spülkappe entspricht, ausgeblasen oder allgemein verdrängt werden.

In weiteren Beispielen umfasst das Verfahren das Einbringen des zweiten Fluids in die zweite Fluidführung.

In bestimmten Beispielen wird das zweite Fluid zum Ableiten bzw. Abführen des ersten, medizinischen, Fluids aus dem Inneren der Spülkappe eingesetzt.

In solchen Beispielen kann das Verfahren dazu eingesetzt werden, einen zum Beispiel nach dem Reinigen des Konnektors mit Substituatflüssigkeit gefüllten Konnektor zu entleeren. Es kann ferner dazu eingesetzt werden, den nach dem Reinigen durch das Reinigen bedingt noch feuchten Konnektor mittels Luft (oder ganz allgemein) zu trocknen.

In bestimmten Beispielen wird der flüssigkeitsgefüllte Konnektor durch Einleiten von Luft über das zweite Lumen und Ableiten der Flüssigkeit über das dritte Lumen geleert. Dies kann beispielsweise oder vorzugsweise durch Einbringen von Luft und dadurch Verdrängen der Flüssigkeit oder durch Absaugen der Flüssigkeit bei zu einem Äußeren des Konnektors, z.B. zur Atmosphäre, geöffnetem zweiten Lumen erfolgen.

Eine Reinigung des erfindungsgemäßen Konnektors kann erfolgen, indem im Verschlusszustand des Konnektors zunächst das erste, medizinische, Fluid über ein Ventil der ersten Fluidführung in das erste Lumen der ersten Fluidführung eingebracht wird. Nach anschließendem Reinigen des Inneren (kann dem ersten und/oder dem zweiten und/oder dem dritten Lumen entsprechen) und/oder des Äußeren des Konnektors (kann dem Inneren der Spülkappe entsprechen), kann das erste Fluid über ein Ventil der zweiten Fluidführung aus dem zweiten Lumen der zweiten Fluidführung und/oder ein Ventil der dritten Fluidführung aus dem dritten Lumen der dritten Fluidführung ausgetragen werden.

Das Einbringen des ersten Fluids zum Reinigen des Konnektors und das Austragen des ersten Fluids können zu verschiedenen Zeitpunkten erfolgen - eine kontinuierliche Spülung ist jedoch ebenso von vorliegendem Beispiel umfasst.

In bestimmten Beispielen befindet sich der erfindungsgemäße Konnektor nach dem Spülen in einem Grundzustand, in welchem er im Wesentlichen oder vollständig mit erstem Fluid gefüllt ist und bis zur vorgesehenen Konnektion des Konnektors mit der Funktionseinrichtung (zu einem beliebigen Zeitpunkt nach der Reinigung) bleibt.

Das Reinigen des erfindungsgemäßen Konnektors kann vor dem erstmaligen Verbinden des Konnektors mit der Funktionseinrichtung und/oder während bzw. zwischen verschiedenen Behandlungsverfahren erfolgen.

In weiteren Beispielen wird die Luft, welche der Spülkappe über die zweite Fluidführung zugeführt wird, mittels von einer Unterdruck erzeugenden Vorrichtung wie z. B. der Ultrafiltrationspumpe oder der Bilanzkammer usw. erzeugten Unterdrucks aus der Umgebung gewonnen.

In manchen Beispielen weist die Behandlungsvorrichtung ein hierzu nutzbares Ventil auf, welches sich in der pneumatischen Leitung befindet oder mit dieser in Fluidverbindung verbunden ist. Mittels des Ventils ist die pneumatische Leitung zur Umgebung geöffnet oder kann gegenüber der Umgebung geöffnet werden.

Bei dieser Anordnung ist es beispielsweise möglich, Umgebungsluft, welche über einen geeigneten Filter strömen kann, mittels Unterdruck zu gewinnen und durch die Spülkappe hindurchzuführen.

Alle, einige oder manche Schritte eines beispielhaften Verfahrens, wie es anhand der beigefügten Zeichnung beschrieben ist, können automatisch und/oder automatisiert durchgeführt werden.

Ferner wird das Verfahren optional auch umgesetzt durch ein digitales Speichermedium, ein Computer-Programm-Produkt sowie ein Computer-Programm.

Ein digitales Speichermedium, insbesondere ein RAM oder ROM oder eine Diskette, CD oder DVD, mit elektrisch auslesbaren Steuersignalen kann derart mit einem programmierbaren Computersystem zusammenwirken, dass die maschinellen Schritte eines beispielhaften Verfahrens veranlasst werden.

Dabei können alle, einige oder manche der maschinell durchgeführten Schritte des beispielhaften Verfahrens veranlasst werden.

Ein Computer-Programm-Produkt weist einen auf einem maschinenlesbaren Träger gespeicherten Programmcode zur Veranlassung der maschinellen Schritte des beispielhaften Verfahrens, wenn das Computer-Programm-Produkt auf einem Rechner abläuft, auf.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein Computer-Programm weist einen Programmcode auf zur Veranlassung der maschinellen Schritte eines beispielhaften Verfahrens, wenn das Computer-Programm auf einem Computer abläuft.

Auch für das Computer-Programm-Produkt und das Computer-Programm gilt, dass alle, einige oder manche der maschinell durchgeführten Schritte des beispielhaften Verfahrens veranlasst werden können.

Bestimmte erfindungsgemäße Ausführungsformen oder beispielhafte Varianten weisen einen oder mehrere der im Folgenden genannten Vorteile auf.

Die vorliegende Erfindung stellt einen Konnektor bereit, welcher vorteilhaft hohen Hygieneansprüchen im Rahmen medizinischer Behandlungsverfahren genügen kann.

So kann es mit dem erfindungsgemäßen Konnektor vorteilhaft möglich sein, selbst in der im oder auf dem Konnektor herrschenden feuchten Umgebung, in welcher gewöhnlich die Gefahr von Keimansammlungen besteht, Keimfreiheit sicherzustellen und damit ein Kontaminationsrisiko für Benutzer und/oder Patienten zu minimieren oder ganz auszuschließen.

Der über die Spülkappe greifende Konnektor, bei welchem wenigstens ein Abschnitt der Spülkappe zwischen einem ersten Teil und einem zweiten Teil des Konnektors angeordnet ist, kann in bestimmten Ausführungsformen der vorliegenden Erfindung vorteilhaft zur vollständigen Entleerbarkeit des Konnektors beitragen. Denn auf diese Weise können besonders geeignete Fluidbahnen innerhalb der Spülkappe verwirklicht werden.

Da die Lufteinleitung der Sterilluft vorteilhaft nicht im Strömungszweig des medizinischen Fluids im ersten Lumen liegt, kann in bestimmten Ausführungsformen der vorliegenden Erfindung vorteilhaft eine Kontamination des medizinischen Fluids vermieden werden.

Eine Keimfreiheit der Stirnfläche der ersten Fluidführung, welche Kontakt z.B. zu der angesaugten Luft als zweitem Fluid hat, kann in bestimmten Ausführungsformen der vorliegenden Erfindung vorteilhaft durch die Verwendung von Sterilluft sichergestellt werden.

Das Leeren des erfindungsgemäßen Konnektors mittels Sterilluft vor dessen Anschließen an die Funktionseinrichtung kann somit in bestimmten Ausführungsformen der vorliegenden Erfindung vorteilhaft sicherstellen, dass die zur Fluidverbindung vorgesehenen Fluidführungen und Kontaktflächen des Konnektors von möglichen Keimen gereinigt, d.h. "freigeblasen" sind.

Auf diese Weise kann es vorteilhaft möglich sein, das Eintragen ggf. kontaminierender Partikel und Keime in die Funktionseinrichtung zu verhindern und damit vorteilhaft einer möglichen Infektion eines mit der Funktionseinrichtung zum Zwecke einer Behandlung verbundenen Patienten vorzubeugen.

Daneben kann, da das Öffnen des erfindungsgemäßen Konnektors unter Überdruck erfolgt, in bestimmten Beispielen ferner ein Verschleppen von freien Partikeln in das erste, zur Fluidverbindung mit der Funktionseinrichtung vorgesehene Lumen verhindert werden. Daneben kann der Einsatz von Überdruck in bestimmten Beispielen vorteilhaft dazu beitragen, ein Einbringen bzw. Eintragen von Kontaminationen aus einem Dichtbereich zwischen Spülkappe und Konnektor in die sterile Zone zu vermeiden, was bei Öffnung eines Konnektors ohne Überdruck und einem ggf. entstehenden Unterdruck passieren könnte.

Ferner kann der erzeugte Überdruck in bestimmten Beispielen vorteilhaft dazu beitragen, Flüssigkeitsreste (z.B. vom letzten Verschlussvorgang) durch den entweichenden Überdruck nach außen wegzuschleudern.

Eine hydrophile Beschichtung des Konnektors, insbesondere im oder am Endabschnitt desselben, kann ferner vorteilhaft dazu beitragen, ein Wegfliegen von Flüssigkeitströpfchen beim Öffnen des Konnektors zu verhindern.

Der Überdruck kann ferner dazu beitragen, die zum Trennen des Konnektors von der Spülkappe aufzubringenden Kräfte vorteilhaft zu reduzieren.

Insbesondere in Ausführungsformen, in denen der Konnektor für einen Benutzer nicht zugänglich in einem Inneren einer Behandlungsvorrichtung angeordnet ist, so dass ein regelmäßiges Abwischen des Konnektors daher nicht möglich ist, kann mit dem erfindungsgemäßen Konnektor vorteilhaft eine saubere und hygienische Verbindung zur Funktionseinrichtung sichergestellt werden.

Im Folgenden wird die vorliegende Erfindung unter Bezugnahme auf die beigefügten Figuren beschrieben. In der Zeichnung bezeichnen identische Bezugszeichen gleiche oder identische Elemente. Es gilt:
- Fig. 1: zeigt schematisch einen erfindungsgemäßen Konnektor zur Verbindung mit einer medizintechnischen Funktionseinrichtung; und
- Fig. 2: zeigt schematisch vereinfacht einen Fluidleitungsaufbau einer erfindungsgemäßen medizintechnischen Behandlungsvorrichtung und einer medizintechnischen Funktionseinrichtung.

**Fig. 1** zeigt schematisch einen erfindungsgemäßen Konnektor 100 in einer beispielhaften Ausführungsform.

Fig. 1 zeigt den Konnektor 100 in einem nicht mit einer medizintechnischen Funktionseinrichtung 1000 konnektierten Zustand. Er ist mit einer Spülkappe 200 verschlossen ("Spülzustand" oder "Verschlusszustand").

Die Spülkappe 200 des Konnektors 100 kann so ausgeführt sein, dass sie in der Spülstellung oder dem Spülzustand zur Außenseite der medizintechnischen Behandlungsvorrichtung 2000 hin abdichtet.

Der Konnektor 100 weist einen mehrlumigen Endabschnitt 1 auf.

Der mehrlumige Endabschnitt 1 des Konnektors 100 weist eine erste Fluidführung 3 mit einem ersten Lumen 31 und einem freien Ende 33 auf. Das erste Lumen 31 ist geeignet und vorgesehen, um Substituatflüssigkeit zu der Funktionseinrichtung 1000, von welcher allein ein Verbindungsabschnitt 1001 eines Substituat-Ports zur Aufnahme des Konnektors 100 gezeigt ist, zu leiten.

Der mehrlumige Endabschnitt 1 des Konnektors 100 weist ferner eine zweite Fluidführung 5 mit einem zweiten Lumen 51 auf. Das zweite Lumen 51 ist geeignet und vorgesehen, Sterilluft von einer in Fig. 1 nicht dargestellten Zuführungseinrichtung, z.B. einem Kompressor, aufzunehmen und über ein freies Ende 53 an einen äußeren Abschnitt des Konnektors 100 und in ein Inneres der Spülkappe 200 zu leiten.

Der mehrlumige Endabschnitt 1 des Konnektors 100 weist eine dritte Fluidführung 7 mit einem dritten Lumen 71 und einem freien Ende 73 auf. Das dritte Lumen 71 ist geeignet und vorgesehen, um sowohl Substituatflüssigkeit als auch Sterilluft zu einem Äußeren des Konnektors 100, z.B. in ein Inneres einer Behandlungsvorrichtung (in Fig. 1 nicht gezeigt) hinein, abzuleiten.

Zu seiner Konnektion oder Dekonnektion mit der Funktionseinrichtung 1000 ist der Konnektor 100 in einer Richtung A der Ankopplung zur Funktionseinrichtung 1000 hin oder von dieser weg verschiebbar. Der Doppelpfeil veranschaulicht die Verschieblichkeit bzw. Beweglichkeit des Konnektors 100.

In der in Fig. 1 gezeigten Ausführung des erfindungsgemäßen Konnektors 100 ist die erste Fluidführung 3 Teil eines Innenrohrabschnitts des Konnektors 100. Die zweite Fluidführung 5 und die dritte Fluidführung 7 sind jeweils Teil eines Außenrohrabschnitts des Konnektors 100.

Die Fluidführungen 3, 5 und/oder 7 können koaxial oder parallel zueinander angeordnet sein wie in Fig. 1, oder nicht. Die Querschnitte der Fluidführungen 3, 5 und/oder 7 können kreisförmig (Bohrungen) sein oder jede andere geeignete Querschnittsform, z. B. ringförmig, aufweisen.

Die geometrische Ausgestaltung des Querschnitts einer Fluidführung 3, 5 oder 7 (bezogen auf einen Querschnitt senkrecht zu einer Durchströmungsrichtung der jeweiligen Fluidführung 3, 5, 7) kann unabhängig von den Querschnitten der anderen Fluidführungen kreisförmig, rechteckig, quadratisch, oval bzw. elliptisch, dreieckig, vieleckig und dergleichen sein, ohne jedoch hierauf beschränkt zu sein.

Die erste Fluidführung 3 weist einen kugelförmigen Endbereich 35 mit einem freien Ende 33 des ersten Lumens 31 auf.

Der Endbereich 35 kann kegelförmig oder in anderer Weise geeignet ausgeführt sein.

In der in Fig. 1 gezeigten Ausführungsform des erfindungsgemäßen Konnektors 100 sind das freie Ende 53 der zweiten Fluidführung 5 und das freie Ende 73 der dritten Fluidführung 7 im Bereich des Endabschnitts 1 des Konnektors 100 gegenüber dem freien Ende 33 der ersten Fluidführung 3 zurückgesetzt, bezogen jeweils auf die Richtung A der Ankopplung.

Die Spülkappe 200 ist derart am Konnektor 100 angeordnet, dass eine Außenseite 201 der Spülkappe 200 zwischen einem radial äußeren Teil 101 und einem radial inneren Teil 103 des Konnektors 100 angeordnet ist.

Ein Dichtring 203 ist als Beispiel einer beliebig ausgestalteten Dichteinrichtung (z.B. Labyrinth usw.) zwischen Spülkappe 200 und dem radial äußeren Teil 101 des Konnektors 100 angeordnet.

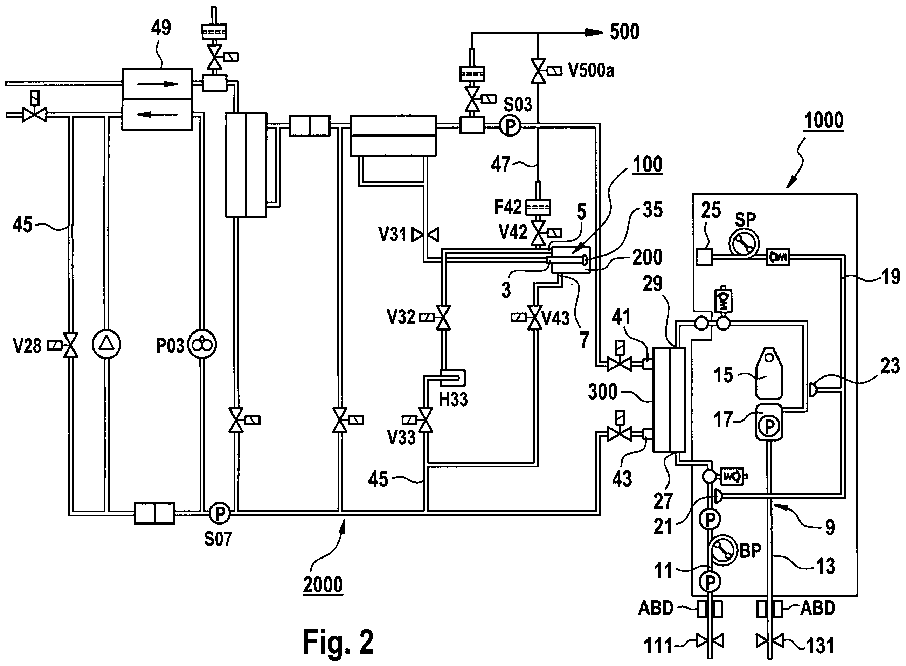

**Fig. 2** zeigt schematisch vereinfacht einen Fluidleitungsaufbau einer medizintechnischen Behandlungsvorrichtung 2000 und einer medizintechnischen Funktionseinrichtung 1000.

Die Funktionseinrichtung 1000 ist in Fig. 2 exemplarisch eine Blutkassette.

Die Blutkassette, hier ausgestaltet als Blutkassette zur Durchführung eines extrakorporalen Blutbehandlungsverfahrens, weist einen extrakorporalen Blutkreislauf 9 mit arteriellem Leitungsabschnitt 11 und venösem Leitungsabschnitt 13, Blutpumpe BP, Single-Needle-Kammer 15, venöser Blutkammer 17 und verschiedenen Drucksensoren P auf. Sensoren ABD ("air bubble detector") zum Detektieren von Luft im extrakorporalen Blutkreislauf sind im arteriellen Leitungsabschnitt 11 und im venösen Leitungsabschnitt 13 angeordnet. Der arterielle Leitungsabschnitt 11 weist ferner eine arterielle Schlauchklemme 111 auf, der venöse Leitungsabschnitt 13 eine venöse Schlauchklemme 131.

Eine Behandlungseinrichtung 300, z.B. eine Dialysiereinrichtung wie ein Blutfilter (Dialysator) zum Durchführen einer Hämodialyse, einer Hämofiltration oder einer Hämodiafiltration, ist mit der Blutkassette - am Bluteinlass 27 und am Blutauslass 29 - und der Behandlungsvorrichtung 2000 - am Dialysateinlass 41 und am Dialysatauslass 43 - verbunden. Das während einer extrakorporalen Blutbehandlung eingesetzte Dialysat kann - gleich der Substituatflüssigkeit - online von der Behandlungsvorrichtung hergestellt werden. Geeignete Einrichtungen zur Filtrierung der Substituatflüssigkeit oder Dialysats, wie beispielsweise so genannte "Online-Filter", können entsprechend vorgesehen sein.

Die Blutkassette weist eine Substituatleitung 19 mit einer Substituatpumpe SP auf.

Während eines extrakorporalen Blutbehandlungsverfahrens kann vorgesehen sein, Substituatflüssigkeit über einen Prädilutionsanschluss 21 und/oder einen Postdilutionsanschluss 23 in den extrakorporalen Blutkreislauf 9 einzubringen.

Der erfindungsgemäße Konnektor 100 ist in der in Fig. 2 gezeigten Ausführungsform Teil der Behandlungsvorrichtung 2000.

In bestimmten Ausführungsformen der vorliegenden Erfindung ist der erfindungsgemäße Konnektor 100 dazu vorgesehen, in einem mit der Funktionseinrichtung 1000 konnektierten Zustand (in Fig. 2 nicht gezeigt) Substituatflüssigkeit von der Behandlungsvorrichtung 2000, insbesondere von der Behandlungsvorrichtung 2000 online hergestellte Substituatflüssigkeit, in die Funktionseinrichtung 1000 einzuleiten. Hierzu wird der Konnektor 100 mit einem Substituat-Port 25 der Funktionseinrichtung 1000 fluidführend verbunden.

Vor Beginn und/oder während eines Behandlungsverfahrens ist es in bestimmten Beispielen vorgesehen, den noch nicht mit der Funktionseinrichtung 1000 konnektierten Konnektor 100 zu reinigen.

Der Konnektor 100 befindet sich hierzu in einem Verschlusszustand. Die Spülkappe 200 ist am Konnektor 100 angeordnet.

Um den erfindungsgemäßen Konnektor 100 von innen und/oder außen vollständig zu reinigen, kann Substituatflüssigkeit von einer Substituatflüssigkeitsquelle (in Fig. 2 nicht gezeigt) in den Konnektor 100 eingebracht werden.

Die Reinigung kann in bestimmten Beispielen der vorliegenden Erfindung über einen ersten Reinigungsweg erfolgen, welcher über ein Ventil V31 der ersten Fluidführung 3, das erste Lumen 31 des Konnektors 100 den Konnektor 100 sowie ein Ventil V43 der dritten Fluidführung 7, in dieser Reihenfolge, verläuft.

In manchen Beispielen kann die Reinigung über einen zweiten Reinigungsweg erfolgen, welcher über das Ventil V31 der ersten Fluidführung 3, den Konnektor 100, ein Ventil V32 der zweiten Fluidführung 5, einen Spülport H33 sowie ggf. ein erstes Ventil V33 einer Drain-Leitung 45, in dieser Reihenfolge, verläuft. Der Spülport H33 kann über Lufteinleitung entlang des Pfads V42 - V32 - H33 - V33 ohne Benutzereingriff geleert werden.

In bestimmten Beispielen kann in der Spülkappe 200 enthaltene Luft über die zweite Fluidführung 5 entweichen; eine entsprechende Entlüftungsmöglichkeit ist in erfindungsgemäßen Ausführungsformen vorgesehen.

Mittels Drucksensoren S03 und/oder S07 kann in Beispielen jeder der vorstehend angegebenen Reinigungswege auf Durchfluss überwacht werden, z.B. durch Einstellen und/oder Auswerten einer Druckdifferenz S03-S07.

In bestimmten Beispielen wird am Ende des Reinigungsverfahrens eine Strecke zwischen einem Ventil V42 einer Sterilluftzuleitung 47, dem Ventil V32 der zweiten Fluidführung 5, dem Spülport H33 sowie dem ersten Ventil V33 der Drain-Leitung 45 durch Aktivierung des an einer Luftverteilerplatte 500 (LVP) angeschlossenen Kompressors entleert. Dies ist jedoch nicht zwingend erforderlich. In bestimmten Beispielen kann dies jedoch insbesondere im Hinblick auf einen ggf. nachfolgend erfolgenden Selbsttest der Blutbehandlungseinrichtung 300 ("T1-Test") von Vorteil sein.

Während der Leerung des Spülports H33 kann die Funktion des ersten Ventils V33 der Drain-Leitung 45 getestet werden.

In bestimmten Beispielen kann es vorteilhaft sein, auch eine Strecke über ein Ventil V43 der dritten Fluidführung 7 am Ende des Reinigungsprogramms zu leeren.

Daneben kann ein trockener Konnektor 100 in bestimmten Ausführungsformen den Vorteil bieten, das Wachstum von hydrophilen Keimen und/oder eines Biofilms zu minimieren oder gar ganz auszuschließen.

Nach Ende des Selbsttests der Blutbehandlungseinrichtung 300 kann die Funktionseinrichtung 1000 automatisch gefüllt und/oder gespült werden.

In bestimmten Beispielen wird hierzu für einen Druckhaltestest die im Filter enthaltene Flüssigkeit verdrängt. Dies kann über eine Strecke Ventil V31 der ersten Fluidführung 3, Konnektor 100 sowie Ventil V43 der dritten Fluidführung 7 erfolgen. In solchen Beispielen wird die Strecke Konnektor 100, Ventil V32 der zweiten Fluidführung 5, Spülport H33, erstes Ventil V33 der Drain-Leitung 45 nicht verwendet. Der Spülport H33 kann so vorteilhaft schon im Selbsttest der Blutbehandlungseinrichtung 300 aufgerüstet werden.

Im Rahmen des Selbsttests der Blutbehandlungseinrichtung 300 wird in einem Beispiel die Strecke V32 - V33 nicht benötigt. Daher kann ein Spülstecker der medizintechnischen Funktionseinrichtung 1000, welcher zum Abführen der gebrauchten Spüllösung aus der medizintechnischen Funktionseinrichtung 1000 dient und beim Füllen/Spülen mit dem venösen Leitungsabschnitt 13 verbunden ist, bereits gesteckt werden, obwohl hierdurch die Flüssigkeitsverbindung zwischen dem Ventil V32 der zweiten Fluidführung 5 und dem ersten Ventil V33 der Drain-Leitung 45 flüssigkeitsdicht verschlossen wird. Dies war bisher so nicht möglich, da die Strecke V32 - V33 in den bisherigen Implementierungen zum Füllen der Substituatstrecke zwingend benötigt wurde.

Durch Verwenden des erfindungsgemäßen Konnektors 100 und/oder der erfindungsgemäßen medizintechnischen Behandlungsvorrichtung 2000 und/oder der erfindungsgemäßen medizintechnischen Funktionseinrichtung 1000 kann somit vorteilhaft vermieden werden, den Anwender der Blutbehandlungseinrichtung 300 nach Ende des Selbsttests der Blutbehandlungseinrichtung 300 bzw. deren Vorbereitung auf die Behandlung zum Starten der Behandlung nochmals an diese zu rufen, wie es bislang erforderlich war. Das Füllen/Spülen der erfindungsgemäßen medizintechnischen Funktionseinrichtung 1000 kann nun vorteilhaft automatisch gestartet werden.

Damit ist es nun vorteilhaft möglich, die Blutbehandlungseinrichtung 300 ohne Benutzerinteraktionen einzuschalten und für die Blutbehandlung vorzubereiten. Benutzereingriffe sind erst mit dem Anschluss des Patienten erforderlich. Dies kann vorteilhaft dazu beitragen, Arbeitsaufwand und Zeit einzusparen.

Durch die vorangegangene Leerung tritt keine Flüssigkeit bei der Konnektion von Konnektor 100 mit dem Substituat-Port 25 der Funktionseinrichtung 1000 aus.

Der Konnektor 100 und die Funktionseinrichtung 1000 werden vor Beginn einer Behandlung in bestimmten Ausführungsformen nach Ende des Selbsttests oder T1-Tests der Hydraulik miteinander verbunden.

Bevor der Konnektor 100 die Spülkappe 200 verlässt, wird die Spülkappe 200 geleert. In bestimmten Beispielen kann dies im Rahmen des Selbsttests der Blutbehandlungseinrichtung 300 erfolgen.

Zum Entleeren der Spülkappe 200 wird über das Ventil V42 der Sterilluftzuleitung 47 Luft in die zweite Fluidführung 5 eingeblasen. Das Entleeren der Spülkappe 200 erfolgt beispielsweise unmittelbar nach dem Selbsttest der medizintechnischen Behandlungsvorrichtung 2000, wenn die medizintechnische Funktionseinrichtung 1000 an der medizintechnischen Behandlungsvorrichtung 2000 aufgerüstet ist.

Die eingeblasene Luft wird in bestimmten Beispielen über einen Sterilfilter F42 vor dem Ventil V42 der zweiten Fluidführung 5 sterilfiltriert. Der Sterilfilter F42 ist, wie in Fig. 2 gezeigt, mit der Eintrittsöffnung des zweiten Lumens verbunden.

Die in die zweite Fluidführung 5 eingeblasene Sterilluft wird am freien Ende der zweiten Fluidführung 5 aus dieser ausgeblasen und verdrängt die im Konnektor 100 bzw. der Spülkappe 200 befindliche Substituatflüssigkeit über das Ventil V43 der dritten Fluidführung 7.

Die eingebrachte Luftmenge kann über verschiedene Mechanismen sichergestellt werden, z.B. über eine Zeitsteuerung des Ventils V42 der Sterilluftzuleitung 47 und Verdrängung - z.B. über ein zweites Ventil V28 der Drain-Leitung 45 - mit ggf. gleichzeitiger Überwachung des Druckverlaufs am Drucksensor S07 und/oder über eine bilanzierte Entfernung der Substituatflüssigkeit z.B. mittels einer Bilanzkammer 49 und/oder einer Ultrafiltrationspumpe P03 und/oder über eine Überwachung z.B. eines Signals eines Blutleck-/Trübungssensors bei Durchgang einer Flüssigkeits-/Luft-Grenzfläche.

Da der Konnektor 100 in bestimmten Ausführungsformen der vorliegenden Erfindung zur Horizontalen geneigt angeordnet ist, so dass dessen freies Ende 33 an der tiefsten Stelle des Lumens der Spülkappe 200 liegt, kann die Spülkappe 200 vorteilhaft nahezu komplett entleert werden.

Während der Leerung des Konnektors 100 ist das Ventil V31 der ersten Fluidführung 3 geschlossen. In bestimmten Beispielen wird somit vorteilhaft ein Eindringen von Luft in die sterile erste Fluidführung 3, welche zur Leitung von steriler Substituatflüssigkeit in die Funktionseinrichtung 1000 während der Behandlung vorgesehen ist, verhindert.

Daneben wird vor Öffnen des Ventils V42 der Sterilluftzuleitung 47 vorteilhaft sichergestellt, dass ein Druckgefälle zu einer Strömung der Substituatflüssigkeit in Richtung der Drain-Leitung 45 führt. In manchen Beispielen kann die Strecke Ventil V42 der Sterilluftzuleitung 47 und Sterilluftfilter F42 somit vorteilhaft vor eindringender Substituatflüssigkeit geschützt werden.

Wenn die Spülkappe 200 geleert wurde, wird in bestimmten Beispielen durch Schließen des Ventils V31 der ersten Fluidführung 3, des Ventils V32 der zweiten Fluidführung 5 und des Ventils V43 der dritten Fluidführung 7 ein Überdruck im Konnektor 100 unter Wirkung der Spülkappe 200 erzeugt.

Um den Konnektor 100 mit der Funktionseinrichtung 1000 zu konnektieren, wird der Konnektor 100 aus der Spülkappe 200 bewegt.

Eine Dichtung zwischen Konnektor 100 und Funktionseinrichtung 1000 bzw. dem Substituat-Port 25 der Funktionseinrichtung 1000 in Konnektionsstellung wird in bestimmten Ausführungsformen durch den kugelförmigen Endbereich 35 der ersten Fluidführung 3 in der Substituatleitung 19 der Funktionseinrichtung 1000 realisiert.

Zu Beginn des Füllens kann diese Verbindung auf Dichtigkeit getestet werden. Sollte während des Füllens bzw. während der Behandlung Leckage auftreten, so kann diese zu einem Leckagesensor in der Behandlungsvorrichtung 2000 abgeführt werden.

Nach Beenden einer Behandlung, kann der Substituat-Port 25 der Funktionseinrichtung 1000 verschlossen werden. Auf diese Weise kann es vorteilhaft möglich sein, ein Heraustropfen von ggf. kontaminierter Flüssigkeit während oder nach dem Abrüsten der Behandlungsvorrichtung 2000 mit der Funktionseinrichtung 1000 zu verhindern.

Ein beispielhaftes Verfahren zum Verschließen der Funktionseinrichtung 1000 bzw. des Substituat-Ports 25 der Funktionseinrichtung 1000 ist in der am 23. Juli 2010 von der Anmelderin unter dem Amtszeichen DE 10 2010 032 181.8 beim Deutschen Patent- und Markenamt hinterlegten Anmeldung mit dem Titel *"Ankoppeleinrichtung, Konnektor, medizintechnische Funktionseinrichtung, medizintechnische Behandlungsvorrichtung sowie Verfahren"* beschrieben.

Nach Rückkehr des Konnektors 100 in eine Verschluss- bzw. Spülstellung kann die Funktionseinrichtung 1000 abgerüstet, d.h. von der Behandlungsvorrichtung 2000 dekonnektiert, werden. Der Konnektor 100 kann erneut gereinigt werden.

Nach Ablauf einer Behandlung an der medizintechnischen Behandlungsvorrichtung 2000 werden die wiederverwendbaren Bauteile i. d. R. desinfiziert: Der Konnektor 100 wird hierbei in bestimmten Beispielen der vorliegenden Erfindung mittels Desinfektionsflüssigkeit gereinigt und/oder desinfiziert.

Während des Desinfektionsprogramms der Behandlungsvorrichtung 2000 befindet sich der Konnektor 100 in der Spülstellung.

Alle Flusswege des Konnektors 100 werden wechselweise oder gleichzeitig durchströmt, wobei die Zufuhr einer Desinfektionslösung beispielsweise oder immer oder meist über die erste Fluidführung 3 des Konnektors 100 (über das Ventil V31 der ersten Fluidführung 3) erfolgt.

Die abführenden Flusswege über das Ventil V32 der zweiten Fluidführung 5 und/oder das Ventil V43 der dritten Fluidführung 7 werden - vorzugsweise wechselweise - durchströmt.

Die in die medizintechnische Funktionseinrichtung 1000 eindringende Spitze des Konnektors 100 wird während der Reinigungsprogramme, insbesondere vollständig, mit Desinfektionsflüssigkeit umspült.

Nach Ende des Reinigungsprogramms ist der Konnektor 100, insbesondere vollständig oder im Wesentlichen, mit RO-Wasser (reverse osmosis, RO) gefüllt.

Am Ende des Reinigungsprogramms wird die im Spülport H33 befindliche Luft über das Ventil V42 der Sterilluftzuleitung 47 und das zweite Ventil V28 der Drain-Leitung 45 zum Abfluss verdrängt.

Anschließend wird die Dichtigkeit des Ventils V42 der Sterilluftzuleitung 47, insbesondere nur grob, getestet. Die Dichtigkeit des Ventils V42 kann zusätzlich vor dem Ansaugen der Desinfektionsflüssigkeit und nach dem erfolgten Spülen überprüft werden.

Der Konnektor 100 wird nun mit Substituat gefüllt.

Das nach Ablauf des z. B. wie vorstehend beschriebenen Reinigungsprogramms im Konnektor 100 befindliche Substituat wird im Rahmen des T1-Tests durch Substituat mit physiologischer Zusammensetzung ersetzt.

Wenn die maschinenseitige Substituatleitung einschließlich des Konnektors 100 mit Substituat gefüllt ist, wird der Außenbereich des Konnektors 100 oder das Innere der Spülkappe 200 geleert.

Ein Entleeren des Außenbereichs des Konnektors 100 oder des Inneren der Spülkappe 200 kann vorteilhaft dazu beitragen, dass bei der folgenden Konnektion mit der medizintechnischen Funktionseinrichtung 1000 keine Flüssigkeit aus dem Konnektor 100 austritt oder in einen den Konnektor 100 umgebenden Bereich der Maschine oder in das Gehäuse eintritt. Auf diese Weise kann es vorteilhaft möglich sein, Verkrustungen und/oder Keimwachstum insbesondere im Dichtbereich zwischen dem Konnektor 100 und der Spülkappe 200 zu vermindern oder zu verhindern.

Das Entleeren des Konnektors 100 erfolgt durch Einbringen von Luft über einen Sterilfilter und das Ventil V42 in das Außenlumen oder die zweite oder dritte Fluidführung 5, 7 des Konnektors 100. Die einströmende Luft kann durch Unterdruck so stark expandiert werden, dass der Konnektor 100 vor dem Öffnen der Spülkappe 200 weitestgehend geleert ist.

Um eine Kontamination der Hydraulik durch das Entleeren zu vermeiden, ist zwischen Hydraulik und Pneumatik in bestimmten Ausführungsformen ein Hydrophobfilter angeordnet. Dieser hat beispielsweise eine Porengröße ≤ 2µm.

Während der Konnektion der Funktionseinrichtung 1000 mit der Behandlungsvorrichtung 2000 wird mittels der Bilanzkammer 49 ein Unterdruck im Konnektor 100 erzeugt und aufrechterhalten. Sobald der Dichtungszustand zwischen dem Konnektor 100 und der Spülkappe 200 aufgehoben und/oder die Spülkappe 200 geöffnet wird, wird die Restflüssigkeit durch die einströmende Luft aus dem Dichtbereich mitgerissen und abgesaugt. In bestimmten Beispielen erfolgt das Entleeren des Konnektors 100 zeitgleich mit der Konnektion der Funktionseinrichtung 1000 und der Behandlungsvorrichtung 2000.

Nachdem die medizintechnische Funktionseinrichtung 1000 abgerüstet, d. h. von der medizintechnischen Behandlungsvorrichtung 2000 entfernt wurde, kann der Entleerungsprozess für eine neue medizintechnische Funktionseinrichtung 1000 wiederholt werden.

Im Folgenden wird beispielhaft eine Entleerung des Konnektors 100 beschrieben. Zum besseren Verständnis wird auf die Bauelemente der Fig. 1 und 2 Bezug genommen.

Mittels der Ultrafiltrationspumpe P03 wird ein Unterdruck im Bereich zwischen dem Ventil V33 der Drain-Leitung 45, dem Konnektor 100 und dem Ventil V43 der dritten Fluidführung 7 erzeugt.

Anschließend wird das Ventil V42 der Sterilluftzuleitung 47 kurzzeitig geöffnet (das Ventil V43 der dritten Fluidführung 7 ist während dieser Zeit geschlossen). Hierdurch strömt ggf. unter Restdruck stehende Luft aus der Leitung in die Compliance des Konnektors 100 ein. Anschließend wird das Ventil V43 wieder geöffnet und erneut ein Unterdruck angelegt.

Diese Schritte können mehrfach wiederholt werden.

Anschließend wird der Konnektor 100 bei anstehendem Unterdruck geöffnet (die Ultrafiltrationspumpe P03 wird in dieser Zeit anhand des Werts des Drucksensors S07 geregelt). Die Pneumatik kann derart ausgelegt werden, dass die Leitung zur Luftverteilerplatte 500 zur Atmosphäre geöffnet werden kann und/oder ein Überdruck aufgebaut werden kann.

Der gesamte Entleerungsprozess sollte oder muss innerhalb von 2 min abgeschlossen sein. Ansonsten kann - in bestimmten Ausführungsformen - das Entleeren wegen Zeitüberschreitung als fehlgeschlagen gewertet werden.

Der Unterdruck beim Entleeren des Konnektors 100 (P_Absaug = 300 hPa ± 50 hPa) wird vorzugsweise so ausgewählt, dass der Unterdruck auch bei minimalem Umgebungsdruck (P_ambient = 700 hPa) noch sicher erreicht werden kann und dennoch keine Flüssigkeit bei der Leerung austritt. Das abzusaugende Flüssigkeitsvolumen kann 5 ml betragen.

In der oben beschriebenen beispielhaften Variante wird die Luft, welche der Spülkappe 200 über das Ventil V42 der ersten Sterilluftleitung 47 und über die zweite Fluidführung 5 zugeführt wird, mittels eines - beispielsweise von der Ultrafiltrationspumpe P03 erzeugten - Unterdrucks aus der Umgebung gewonnen. Zu diesem Zweck weist die Behandlungsvorrichtung 2000 in manchen Beispielen ein in der Fig. 2 dargestelltes Ventil V500a auf. Das Ventil V500a befindet sich an beliebiger Stelle in der pneumatischen Leitung, welche zum Bezugszeichen 500 führt, oder es ist mit der pneumatischen Leitung in Fluidverbindung verbunden. Mittels des Ventils V500a ist die pneumatische Leitung zur Umgebung geöffnet oder kann gegenüber der Umgebung geöffnet werden. Bei dieser Anordnung ist es möglich, mittels der Ultrafiltrationspumpe P03 - oder einer anderen Einrichtung, welche Unterdruck aufbauen kann, beispielsweise mittels der Bilanzkammer 49 - am Ventil V43 der dritten Ventilführung 7, über welches die der Spülkappe 200 zugeführte Luft wieder aus der Spülkappe 200 herausgeleitet wird, einen Unterdruck anzulegen. Auf diese Weise wird in der hier beschriebenen Variante Luft aus der Umgebung über das mit der Leitung 500 verbundene Ventil V500a, durch den Sterilluftfilter F42 und durch das Ventil V42 hindurch, aus dem freien Ende 53 der zweiten Fluidführung 5 heraus, durch die Spülkappe 200 hindurch, in das freie Ende 73 der dritten Fluidführung 7 hinein und schließlich durch das Ventil V43 hindurch gesaugt.

**Bezugszeichenliste**

| **Bezugszeichen** | **Beschreibung** |
|---|---|
| 1000 | medizintechnische Funktionseinrichtung |
| 1001 | Verbindungsabschnitt |
| 2000 | medizintechnische Behandlungsvorrichtung |
| 100 | Konnektor |
| 101 | radial äußerer Teil des Konnektors 100 |
| 103 | radial innerer Teil des Konnektors 100 |
| 200 | Spülkappe |
| 201 | Außenseite der Spülkappe 200 |
| 203 | Dichtring |
| 300 | Behandlungseinrichtung |
| 500 | Luftverteilerplatte |
| 1 | mehrlumiger Endabschnitt des Konnektors 100 |
| 3 | erste Fluidführung |
| 31 | erstes Lumen, der ersten Fluidführung 3 |
| 33 | freies Ende der ersten Fluidführung 3 |
| 35 | kugelförmiger Endbereich der ersten Fluidführung 3 |
| 5 | zweite Fluidführung |
| 51 | zweites Lumen, der zweiten Fluidführung 5 |
| 53 | freies Ende der zweiten Fluidführung 5 |
| 7 | dritte Fluidführung |
| 71 | drittes Lumen, der dritten Fluidführung 7 |
| 73 | freies Ende der dritten Fluidführung 7 |
| 9 | extrakorporaler Blutkreislauf |
| 11 | arterieller Leitungsabschnitt |
| 111 | arterielle Schlauchklemme |
| 13 | venöser Leitungsabschnitt |
| 131 | venöse Schlauchklemme |
| 15 | Single-Needle-Kammer |
| 131 | venöse Schlauchklemme |
| 15 | Single-Needle-Kammer |
| 17 | venöse Blutkammer |
| 19 | Substituatleitung |
| 21 | Prädilutionsanschluss |
| 23 | Postdilutionsanschluss |
| 25 | Substituat-Port der Funktionseinrichtung 1000 |
| 27 | Bluteinlass der Funktionseinrichtung 1000 |
| 29 | Blutauslass der Funktionseinrichtung 1000 |
| 41 | Dialysateinlass der Funktionseinrichtung 1000 |
| 43 | Dialysatauslass der Funktionseinrichtung 1000 |
| 45 | Drain-Leitung |
| 47 | Sterilluftzuleitung |
| 49 | Bilanzkammer |
| A | Richtung der Ankopplung |
| ABD | Sensor zum Detektieren von Luft ("air bubble detector") |
| BP | Blutpumpe der Funktionseinrichtung 1000 |
| F42 | Sterilluftfilter |
| H33 | Spülport |
| P | Drucksensoren |
| P03 | Ultrafiltrationspumpe |
| SP | Substituatpumpe der Funktionseinrichtung 1000 |
| S03, S07 | Drucksensoren der Behandlungsvorrichtung 2000 |
| V28 | zweites Ventil der Drain-Leitung 45 |
| V31 | Ventil der ersten Fluidführung 3 |
| V32 | Ventil der zweiten Fluidführung 5 |
| V33 | erstes Ventil der Drain-Leitung 45 |
| V42 | Ventil der Sterilluftzuleitung 47 |
| V43 | Ventil der dritten Fluidführung 7 |
| V500a | Ventil der pneumatisch Leitung zur Umgebung |

## Patentansprüche

1. Konnektor (100) mit Spülkappe (200) zum Herstellen einer Fluidverbindung zwischen wenigstens zwei medizintechnischen Systemen, nachdem der Konnektor (100) aus der Spülkappe (200) bewegt worden ist, aufweisend einen mehrlumigen Endabschnitt (1), welcher wenigstens aufweist:
- eine erste Fluidführung (3) mit wenigstens einem ersten Lumen (31) zum Verbinden der wenigstens zwei medizintechnischen Systeme , vorgesehen zur Aufnahme und/oder Führung eines ersten, medizinischen, Fluids; und
- eine zweite Fluidführung (5) mit wenigstens einem zweiten Lumen (51), vorgesehen zur Aufnahme und/oder Führung eines zweiten, vom ersten verschiedenen, Fluids; und ferner
- eine dritte Fluidführung (7) mit wenigstens einem dritten Lumen (71), vorgesehen zur Aufnahme und/oder Führung des ersten und/oder des zweiten Fluids;
**dadurch gekennzeichnet, dass** der Konnektor (100) vorgesehen und ausgestaltet ist, um in einem Verschlusszustand im Bereich seines Endabschnitts (1) mit der Spülkappe (200) verschlossen zu werden, wobei die Lumen (31, 51, 71) des Konnektors (100) im Verschlusszustand in Fluidkontakt mit einem Inneren der Spülkappe (200) stehen,
wobei die Spülkappe (200) dazu ausgelegt ist, den Konnektor (100) nach außen zu verschließen oder abzudichten,
wobei der Konnektor (100) dazu geeignet ist, dass das zweite Fluid aus der Austrittsöffnung oder dem freien Ende (53) des zweiten Lumens (51) aus dem Konnektor (100) heraus und in das Innere der Spülkappe (200) eingebracht werden kann und
der Konnektor (100) zudem dazu geeignet ist, dass das erste und/oder das zweite Fluid aus dem Inneren der Spülkappe (200) über das dritte Lumen (71) aus dem Konnektor (100) abgeleitet werden kann.

2. Konnektor (100) nach Anspruch 1, wobei die zweite Fluidführung (5) als Zuleitung für das zweite Fluid und die dritte Fluidführung als Ableitung für das erste und/oder das zweite Fluid vorgesehen und ausgestaltet sind, oder umgekehrt.

3. Konnektor (100) nach Anspruch 1 oder 2, wobei die erste Fluidführung (3) Teil eines Innenrohrabschnitts des Konnektors (100) und wobei die zweite und dritte Fluidführung (5, 7) jeweils Teil eines Außenrohrabschnitts des Konnektors (100) sind.

4. Konnektor (100) nach einem der vorangegangenen Ansprüche, wobei die Spülkappe (200) lösbar mit dem Konnektor (100) verbindbar ausgestaltet ist.

5. Konnektor (100) nach einem der vorangegangenen Ansprüche, wobei die Spülkappe (200) automatisch oder automatisiert vom mehrlumigen Endabschnitt verschwenkbar und/oder verschiebbar ausgestaltet ist.

6. Konnektor (100) nach einem der vorangegangenen Ansprüche, wobei die zweite Fluidführung (5) in einem Gebrauchszustand des Konnektors (100) oberhalb der ersten Fluidführung (3) angeordnet ist und/oder wobei die dritte Fluidführung (7) in einem Gebrauchszustand des Konnektors (100) unterhalb der ersten und/oder der zweiten Fluidführung angeordnet ist.

7. Konnektor (100) nach einem der vorangegangenen Ansprüche, wobei ein freies Ende (53) der zweiten Fluidführung (5) als Austrittsöffnung für das zweite Fluid und ein freies Ende (73) der dritten Fluidführung (7) als Eintrittsöffnung für das erste, medizinische Fluid und/oder das zweite Fluid vorgesehen und ausgestaltet ist, oder umgekehrt.

8. Konnektor (100) nach einem der vorangegangenen Ansprüche, wobei die Spülkappe (200) derart am Konnektor (100) angeordnet ist, dass im Zustand der aufgesetzten Spülkappe (200) wenigstens ein Abschnitt der Spülkappe (200) zwischen einem ersten Teil (101) und einem zweiten Teil (103) des Konnektors (100) angeordnet ist.

9. Konnektor (100) nach Anspruch 8, wobei der Abschnitt der Spülkappe (200) zwischen einem radial weiter außen gelegenen Teil und einem radial weiter innen gelegenen Teil des Konnektors (100) angeordnet ist.

10. Konnektor (100) nach einem der vorangegangenen Ansprüche, wobei der Konnektor (100) wenigstens abschnittsweise hydrophil ausgestaltet ist.

11. Konnektor (100) nach einem der vorangegangenen Ansprüche, verbunden mit einer medizintechnischen Behandlungsvorrichtung (2000).

12. Konnektor (100) nach Anspruch 11, wobei die medizintechnische Behandlungsvorrichtung (2000) eine Einrichtung zum Einleiten von Luft in die zweite Fluidführung (5) aufweist.

13. Konnektor (100) nach einem der vorangegangenen Ansprüche, wobei der Konnektor (100) an der medizintechnischen Behandlungsvorrichtung (2000) zur Horizontalen geneigt angebracht ist.

14. Konnektor (100) nach einem der vorangegangenen Ansprüche, verbunden mit einer medizintechnischen Funktionseinrichtung (1000).

15. Konnektor (100) nach Anspruch 14, wobei der Konnektor (100) zur Funktionseinrichtung (1000) hin und von dieser weg beweglich ausgestaltet ist.

## Claims

1. A connector (100) with a rinsing cap (200) for establishing a fluid connection between at least two medical-technical systems, after the connector (100) has been moved out of the rinsing cap (200), comprising a multi-lumen end section (1), which comprises at least:
- a first fluid guide (3) comprising at least a first lumen (31) for connecting the at least two medical-technical systems, provided for receiving and/or guiding a first medical fluid; and
- a second fluid guide (5) comprising at least a second lumen (51), provided for receiving and/or guiding a second fluid being different from the first one; and further
- a third fluid guide (7) comprising at least a third lumen (71), provided for receiving and/or guiding the first and/or the second fluid;
**characterized in that** the connector (100) is provided and designed to be closed in a locking status in the area of its end section (1) with the rinsing cap (200), wherein the lumens (31, 51, 71) of the connector (100) in the locking status are in fluid contact with an inside of the rinsing cap (200),
wherein the rinsing cap (200) is adapted to close or seal the connector (100) to the outside,
wherein the connector (100) is adapted so that the second fluid can be introduced from the outlet opening or from the free end (53) of the second lumen (51) out of the connector (100) and into the inside of the rinsing cap (200) and
the connector (100) is moreover adapted so that the first and/or the second fluid can be derived from the inside of the rinsing cap (200) via the third lumen (71) from the connector (100).

2. The connector (100) according to the first claim, wherein the second fluid guide (5) is provided and designed as a supply line for the second fluid and wherein the third fluid guide is provided and designed as an evacuation line for the first and/or the second fluid, or vice versa.

3. The connector (100) according to claim 1 or 2, wherein the first fluid guide (3) is part of an inner tube section of the connector (100) and wherein the second and third fluid guides (5, 7) are each part of an outer tube section of the connector (100).

4. The connector (100) according to anyone of the preceding claims, wherein the rinsing cap (200) is designed to be detachably connectable with the connector (100).

5. The connector (100) according to anyone of the preceding claims, wherein the rinsing cap (200) is designed to be able to rotate and/or to be moved automatically or in an automated manner from the multi-lumen end section.

6. The connector (100) according to anyone of the preceding claims, wherein the second fluid guide (5) is arranged, in use condition of the connector (100), above the first fluid guide (3) and/or wherein the third fluid guide (7) is arranged, in use condition of the connector (100), below the first and/or the second fluid guide.

7. The connector (100) according to anyone of the preceding claims, wherein a free end (53) of the second fluid guide (5) is provided and designed as an outlet opening for the second fluid and a free end (73) of the third fluid guide (7) is provided and designed as an inlet opening for the first medical fluid and/or for the second fluid, or vice versa.

8. The connector (100) according to anyone of the preceding claims, wherein the rinsing cap (200) is arranged at the connector (100) so that when the rinsing cap (200) is put on, at least one portion of the rinsing cap (200) is arranged between a first part (101) and a second part (103) of the connector (100).

9. The connector (100) according to claim 8, wherein the portion of the rinsing cap (200) is arranged between a part located radially further outside and a part located radially more inside of the connector (100).

10. The connector (100) according to anyone of the preceding claims, wherein the connector (100) is designed at least partially hydrophilic.

11. The connector (100) according to anyone of the preceding claims, which is connected to a medical-technical treatment apparatus (2000).

12. The connector (100) according to claim 11, wherein the medical-technical treatment apparatus (2000) comprises a device for introducing air into the second fluid guide (5).

13. The connector (100) according to anyone of the preceding claims, wherein the connector (100) is mounted horizontally inclined to the medical treatment apparatus (2000).

14. The connector (100) according to anyone of the preceding claims, which is connected to a medical-technical functional device (1000).

15. The connector (100) according to claim 14, wherein the connector (100) is designed to be able to move closer and away from the functional device (1000).

## Revendications

1. Un connecteur (100) avec un bouchon de rinçage (200) pour établir une connexion de fluide entre au moins deux systèmes médico-techniques, après que le connecteur (100) ait été déplacé hors du bouchon de rinçage (200), comprenant une extrémité (1) à canaux multiples qui comprend au moins:
- une première conduite pour fluide (3) comprenant au moins un premier canal (31) pour connecter au moins les deux systèmes médico-techniques, prévue pour recevoir et/ou guider un premier fluide médical; et
- une seconde conduite pour fluide (5) comprenant au moins un second canal (51), prévue pour recevoir et/ou guider un second fluide différent du premier; et de plus
- une troisième conduite pour fluide (7) comprenant au moins un troisième canal (71), prévue pour recevoir et/ou guider le premier et/ou le second fluide;
**caractérisé en ce que** le connecteur (100) est prévu et conçu, en position de verrouillage, de manière à être verrouillé, dans la zone de son extrémité (1), avec le bouchon de rinçage (200), les canaux (31, 51, 71) du connecteur (100) en position de verrouillage étant en contact fluidique avec un intérieur du bouchon de rinçage (200),
le bouchon de rinçage (200) étant apte à verrouiller ou à étanchéifier le connecteur (100) par rapport à l'extérieur,
le connecteur (100) étant apte à ce que le second fluide puisse être introduit, depuis un orifice de sortie ou depuis l'extrémité libre (53) du second canal (51), hors du connecteur (100) et vers l'intérieur du bouchon de rinçage (200) et
le connecteur (100) étant en outre apte à ce que le premier et/ou le second fluide puisse(nt) être dérivé(s), depuis l'intérieur du bouchon de rinçage (200) au moyen du troisième canal (71) hors du connecteur (100).

2. Le connecteur (100) selon la première revendication, où la seconde conduite pour fluide (5) est prévue et conçue comme une conduite d'alimentation pour le second fluide et où la troisième conduite pour fluide est prévue et conçue comme une conduite d'évacuation pour le premier et/ou le second fluide, ou inversement.

3. Le connecteur (100) selon la revendication 1 ou 2, où la première conduite pour fluide (3) fait partie d'une section de tube interne du connecteur (100) et où la seconde et la troisième conduites pour fluide (5, 7) font chacune partie d'une section de tube externe du connecteur (100).

4. Le connecteur (100) selon l'une quelconque des revendications précédentes, où le bouchon de rinçage (200) est conçu de manière à pouvoir être connecté au connecteur (100) de façon détachable.

5. Le connecteur (100) selon l'une quelconque des revendications précédentes, où le bouchon de rinçage (200) est conçu de manière à pouvoir pivoter et/ou à être déplacé automatiquement ou de façon automatisée par rapport à l'extrémité à canaux multiples.

6. Le connecteur (100) selon l'une quelconque des revendications précédentes, où la seconde conduite pour fluide (5) est agencée, lors d'une utilisation du connecteur (100), au-dessus de la première conduite pour fluide (3) et/ou où la troisième conduite pour fluide (7) est agencée, lors d'une utilisation du connecteur (100), en-dessous de la première et/ou de la seconde conduite pour fluide.

7. Le connecteur (100) selon l'une quelconque des revendications précédentes, où une extrémité libre (53) de la seconde conduite pour fluide (5) est prévue et conçue comme un orifice de sortie pour le second fluide et une extrémité libre (73) de la troisième conduite pour fluide (7) est prévue et conçue comme un orifice d'entrée pour le premier fluide médical et/ou le second fluide, ou inversement.

8. Le connecteur (100) selon l'une quelconque des revendications précédentes, où le bouchon de rinçage (200) est agencé sur le connecteur (100) de sorte que lorsque le bouchon de rinçage (200) est ainsi placé, au moins une section du bouchon de rinçage (200) est agencée entre une première partie (101) et une seconde partie (103) du connecteur (100).

9. Le connecteur (100) selon la revendication 8, où la section du bouchon de rinçage (200) est agencée entre une partie située radialement plus à l'extérieur et une partie située radialement plus à l'intérieur du connecteur (100).

10. Le connecteur (100) selon l'une quelconque des revendications précédentes, où le connecteur (100) est au moins partiellement hydrophile.

11. Le connecteur (100) selon l'une quelconque des revendications précédentes, qui est connecté à un appareil de traitement médico-technique (2000).

12. Le connecteur (100) selon la revendication 11, où l'appareil de traitement médico-technique (2000) comprend un dispositif pour introduire de l'air dans la seconde conduite pour fluide (5).

13. Le connecteur (100) selon l'une quelconque des revendications précédentes, où le connecteur (100) est fixé en inclinaison horizontale à l'appareil de traitement médico-technique (2000).

14. Le connecteur (100) selon l'une quelconque des revendications précédentes, qui est connecté à un dispositif de fonctionnement médico-technique (1000).

15. Le connecteur (100) selon la revendication 14, où le connecteur (100) est conçu de manière à pouvoir se rapprocher et s'éloigner du dispositif de fonctionnement médico-technique (1000).
